(19) Europäisches Patentamt
European Patent Office
Office européen des brevets

(11) Publication number: **0 639 569 A1**

(12) **EUROPEAN PATENT APPLICATION published in accordance with Art. 158(3) EPC**

(21) Application number: **92905781.8**

(22) Date of filing: **10.03.92**

(86) International application number: **PCT/JP92/00283**

(87) International publication number: **WO 92/15564 (17.09.92 92/24)**

(51) Int. Cl.[6]: **C07D 213/81**, C07D 401/06, C07D 413/06, C07D 413/14, C07D 417/06, C07D 417/14, C07D 401/14

(30) Priority: **11.03.91 JP 70513/91**
**05.07.91 JP 190850/91**

(43) Date of publication of application: **22.02.95 Bulletin 95/08**

(84) Designated Contracting States: **AT BE CH DE DK ES FR GB GR IT LI LU MC NL SE**

(71) Applicant: **NIPPON SODA CO., LTD.**
**2-1, Ohtemachi 2-chome**
**Chiyoda-ku,**
**Tokyo 100 (JP)**

(72) Inventor: **ISHIMITSU, Keiichi, Odawara Research Center**
**Nippon Soda Co., Ltd.**
**345, Aza Yanagimachi**
**Takada, Odawara-shi**
**Kanagawa 250-02 (JP)**
Inventor: **KISHIMOTO, Takashi, Odawara Research Center**
**Nippon Soda Co., Ltd.**
**345, Aza Yanagimachi**
**Takada, Odawara-shi**
**Kanagawa 250-02 (JP)**
Inventor: **YAMADA, Yasuo, Odawara Research Center**
**Nippon Soda Co., Ltd.**
**345, Aza Yanagimachi**
**Takada, Odawara-shi**
**Kanagawa 250-02 (JP)**
Inventor: **YAMADA, Tomio, Odawara Research Center**
**Nippon Soda Co., Ltd.**
**345, Aza Yanagimachi**
**Takada, Odawara-shi**
**Kanagawa 250-02 (JP)**
Inventor: **TAKAKUSA, Nobuo, Odawara Research Center**
**Nippon Soda Co., Ltd.**
**345, Aza Yanagimachi**
**Takada, Odawara-shi**
**Kanagawa 250-02 (JP)**
Inventor: **YAMAMOTO, Atsushi, Odawara Research Center**
**Nippon Soda Co., Ltd.**
**345, Aza Yanagimachi**
**Takada, Odawara-shi**
**Kanagawa 250-02 (JP)**
Inventor: **MITSUI, Jun, Odawara Research Center**
**Nippon Soda Co., Ltd.**
**345, Aza Yanagimachi**
**Takada, Odawara-shi**
**Kanagawa 250-02 (JP)**

(74) Representative: **de Bruijn, Leendert C. et al**
**Nederlandsch Octrooibureau**
**Scheveningseweg 82**
**P.O. Box 29720**
**NL-2502 LS Den Haag (NL)**

(54) **NOVEL HETEROCYCLIC COMPOUND.**

(57) A compound represented by general formula (I), a process for producing the same, and an insecticide

EP 0 639 569 A1

containing the same: wherein Z represents a 5- or 6-membered nitrogenous hetero-cyclic group which may be substituted; X represents carbon, nitrogen, sulfur or oxygen; Y represents $-CR_3=CR_4-CR_5R-$, etc.; $R_2$ represents an electron drawing group except for nitro, cyano and trifluoroacetyl; $R_1$ and $R_3$ to $R_6$ represent each hydrogen, halogen or lower alkyl which may be substituted.

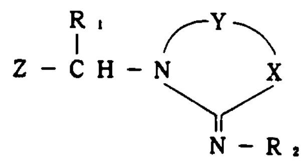

〔 I 〕

Field of the Invention

The present invention relates to novel heterocyclic compounds, a process for the preparation thereof and a insecticide.

Background Art

By virtue of research and development of insecticides for many years, a number of insecticides, for example organophosphorus insecticides such as parathion and malathion, and carbamate insecticides such as carbaryl and methomyl, have been developed and used in practice. The contribution these insecticides to the improvement of agricultural production yield are great.

However, in recent years some of these insecticides have had their use regulated because of problems such as invironmental pollution due to residue or accumulation, or because they cause infestation of resistant insect pests as a result of long-term use. Therefore, the development of novel insecticidal compounds which have excellent insecticidal propterty against insecticide-resistant pest insect species as well as other various pest insects and can be safely used is highly demanded.

As cross references, Japanese Patent Application Laid-Open No.Sho 63-150275, No.Hei 3-190859 which disclosed the compounds having similar structural formula to the heterocyclic compounds of this invention have been known.

It is an object of the present invention to provide an agricultural chemical which can be manufactured advantageously on an industrial scale and can be used safely as well as having firm insecticidal effectiveness.

The present invention is directed at compounds having the general formula [I]:

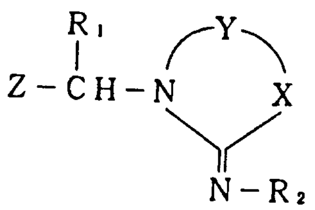

[I]

wherein

Z is an unsubstituted or substituted 5- or 6-membered heterocyclic group containing nitrogen;

X is a carbon, nitrogen, sulfur or oxygen atom;

Y is, when X is a carbon atom, (a) in a form of -Y-X;

(a)

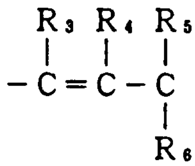

when X is a nitrogen atom, (b) or (c) in a form of -Y-X;

(b)

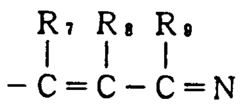

(c)

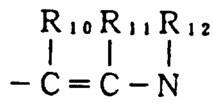

when X is a sulfur atom, (d), (e) or (f) in a form of -Y-X;
(d)

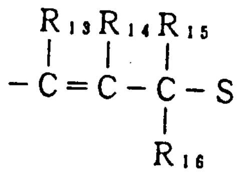

(e)

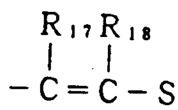

(f)

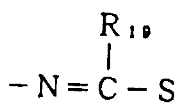

when X is an oxygen atom, (g) or (h) in a form of -Y-X;
(g)

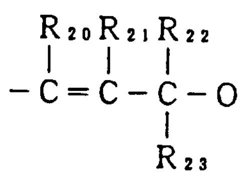

(h)

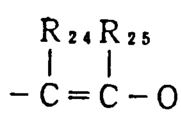 ;

$R_2$ is an electron attractive group other than nitro, cyano and trifluoroacetyl;
$R_1$ and $R_3$ ~ $R_{25}$ are independently a hydrogen atom, a halogen atom or an unsubstituted or substituted lower alkyl;
Or, Y is, when X is a carbon atom, (i) in a form of -Y-X;

(i)

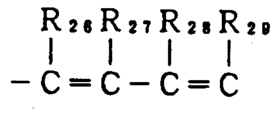 ;

$R_2$ is an unsubstituted or substituted heterocyle carbonyl, other than furancarbonyl, thiophenecarbonyl and pyridinecarbonyl;

$R_{26}$ ~ $R_{29}$ are independently a hydrogen atom, a halogen atom or an unsubstituted or substituted lower alkyl;

and a process for the preparation of the compounds and an insecticide.

Electron attractive groups represented by $R_2$ are for example alkoxycarbonyl including $C_{1-4}$ alkoxycarbonyl such as methoxycarbonyl and ethoxycarbonyl; $C_{6-10}$ arylcarbonyl such as phenylcarbonyl; heterocycle carbonyl such as pyridinecarbonyl, thiophenecarbonyl and pyrazinecarbonyl; $C_{1-4}$ alkylsulfonyl which can be substituted by halogen such as chlorine and bromine, for example, methylsulfonyl, trifluoromethylsulfonyl and ethylsulfonyl; sulfamoyl; $C_{1-4}$ acyl which can be substituted by halogen such as chlorine and bromine, for example, acetyl and trichloroacetyl, and carbamoyl.

## Best Mode for Carrying Out the Invention

The process for preparing the compounds specified in the present invention is as follows.

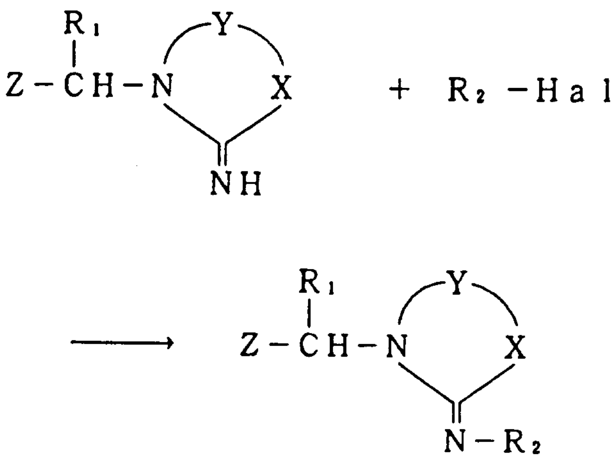

wherein Hal is a halogen atom, Z, X, Y, $R_1$ and $R_2$ are as defined above. Triethylamine, NaH, $Na_2CO_3$ and $K_2CO_3$ etc. are used as an acid acceptor in an inactive solvent such as acetonitrile, methylethylketone, acetone, DMF and the like. The product, in general, can be synthesized by heating at a temperature in the range from room temperature to the boiling point of the solvent used.

The structures of the compounds specified in the present invention were determined based on the analytical results obtained by utilizing IR, NMR, MS and other analytical means.

The compounds specified in the present invention are illustrated in detail by the following Examples.

## Example 1

1-(2-chloro-5-pyridylmethyl)-2-(pyrazinecarbonylimino)-1,2-dihydropyridine (Compound No. 1):

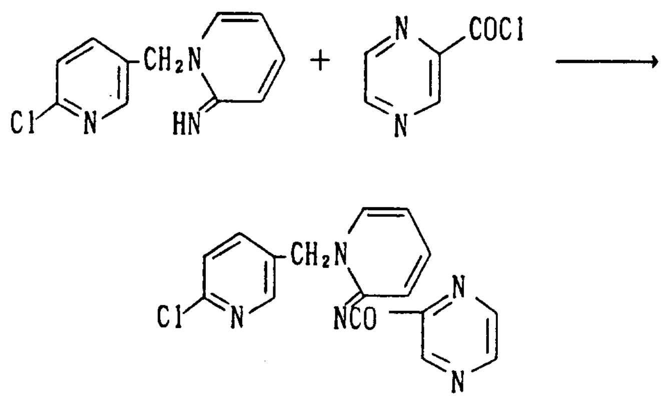

4

1.2 g of pyrazinecarbonyl chloride, and 1.4 g of triethylamine were added to a solution of 1.5 g of 1-(2-chloro-5-pyridylmethyl)-2-imino-1,2-dihydropyridine in 30 ml of acetonitrile.

After refluxing for 3 hours, acetonitrile was distilled off, and the residue was purified by column chromatography to give 1.4 g of the intended product.

Example 2

3-(2-chloro-5-pyridylmethyl)-2-(5-methyl-2-pyrazinecarbonyl-imino)-2,3-dihydrothiazole (Compound No. 152):

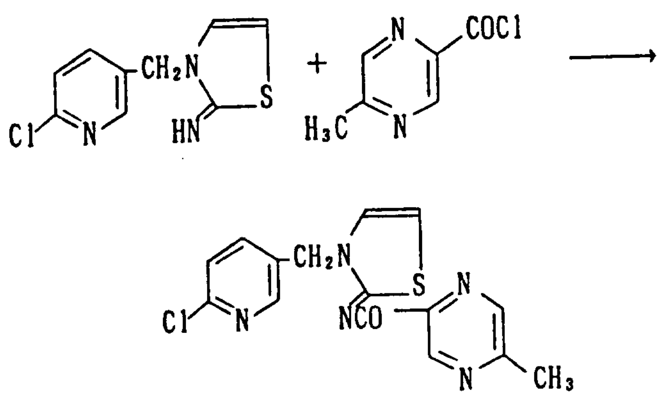

1.1 g of 5-methyl-2-pyrazinecarbonyl chloride, and 0.9 g of triethylamine were added to a solution of 1.5 g of 3-(2-chloro-5-pyridylmethyl)-2-imino-3,3-dihydrothiazole in 30 ml of acetonitrile.

After refluxing for 3 hours, acetonitrile was distilled off, and the residue was purified by column chromatography to give 1.6 g of the intended product. m.p. 205-206 ° C.

The compounds of the present invention are illustrated in Table 1 including the Examples described above.

Table 1

| Compound No. | Structure Formula 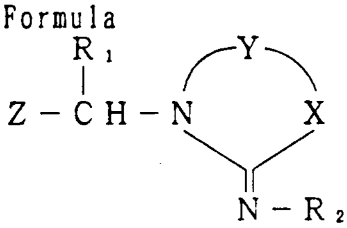 | | | Physical Properties ( ) m.p. ℃ |
|---|---|---|---|---|
| | 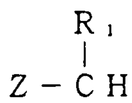 | -Y-X | $R_2$ | |
| 1 | 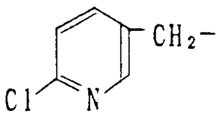 | -CH=CH-CH=CH | 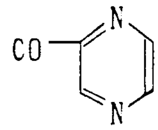 | [194-196] |
| 2 | 〃 | 〃 | 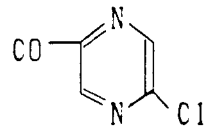 | [207-208] |
| 3 | 〃 | 〃 | 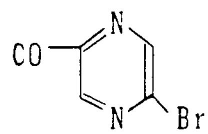 | |
| 4 | 〃 | 〃 | 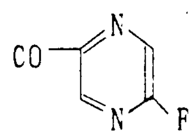 | |
| 5 | 〃 | 〃 | 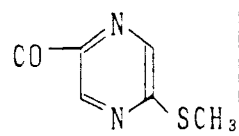 | |
| 6 | 〃 | 〃 | 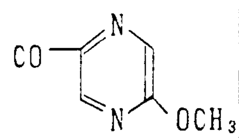 | |

to be continued

| | | | | |
|---|---|---|---|---|
| 7 | 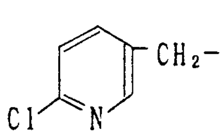 | -CH=CH-CH=CH | 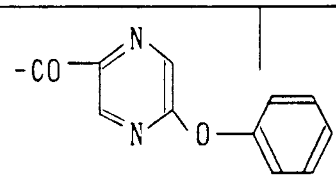 | |
| 8 | 〃 | 〃 | 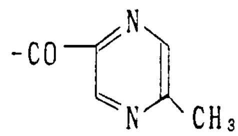 | [220 up] |
| 9 | 〃 | 〃 | 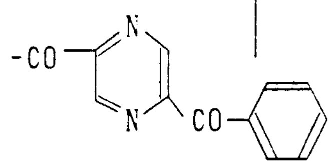 | [148 -150] |
| 10 | 〃 | 〃 | 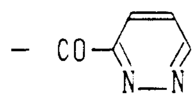 | |
| 11 | 〃 | 〃 | 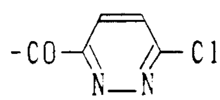 | |
| 12 | 〃 | 〃 | 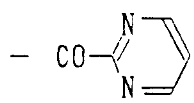 | |
| 13 | 〃 | 〃 | 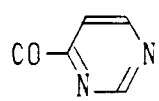 | |
| 14 | 〃 | 〃 | 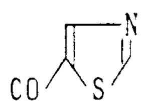 | |
| 15 | 〃 | 〃 | 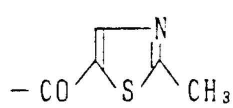 | [158 -159] |
| 16 | 〃 | 〃 | 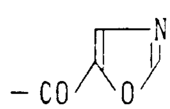 | |

to be continued

| | | | | |
|---|---|---|---|---|
| 17 | 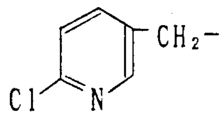 | -CH=CH-CH=CH | 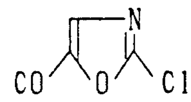 | |
| 18 | 〃 | 〃 | 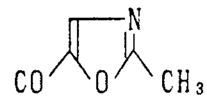 | |
| 19 | 〃 | 〃 | 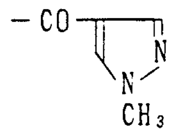 | |
| 20 | 〃 | 〃 | 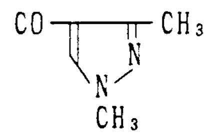 | $n_D^{26}$ 1.6085 |
| 21 | 〃 | 〃 | 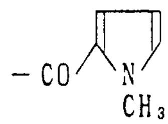 | |
| 22 | 〃 | 〃 | 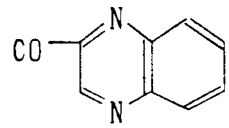 | [218－220] |
| 23 | 〃 | 〃 | 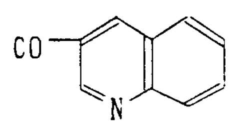 | [172－173] |
| 24 | 〃 | 〃 | 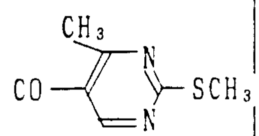 | [151－153] |
| 25 | 〃 | 〃 | 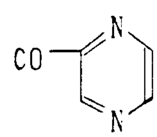 | |

to be continued

| | | | | |
|---|---|---|---|---|
| 26 | 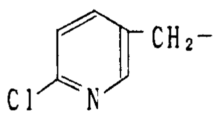 | -CH=CH-CH=CH | 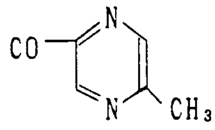 | |
| 27 | ″ | ″ | 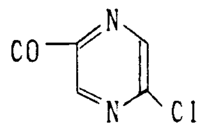 | |
| 28 | ″ | ″ | 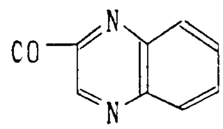 | |
| 29 | ″ | ″ | 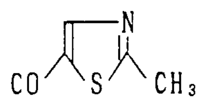 | |
| 30 | 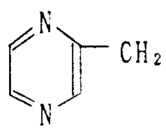 | ″ | 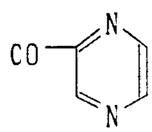 | |
| 31 | ″ | ″ | 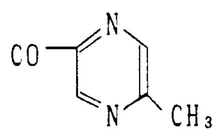 | |
| 32 | ″ | ″ | 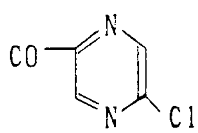 | |
| 33 | ″ | ″ | 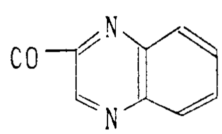 | |
| 34 | ″ | ″ | 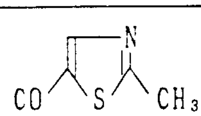 | |

to be continued

| | | | | |
|---|---|---|---|---|
| 35 | 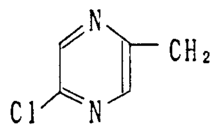 | -CH=CH-CH=CH | 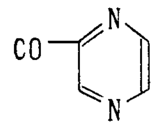 | |
| 36 | 〃 | 〃 | 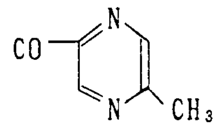 | |
| 37 | 〃 | 〃 | 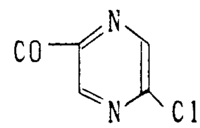 | |
| 38 | 〃 | 〃 | 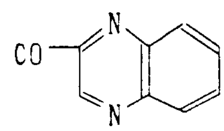 | |
| 39 | 〃 | 〃 | 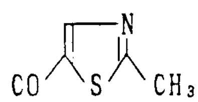 | |
| 40 | 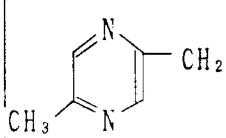 | 〃 | 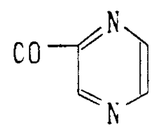 | [185-188] |
| 41 | 〃 | 〃 | 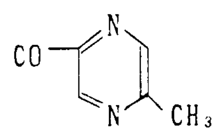 | |
| 42 | 〃 | 〃 | 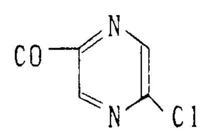 | |
| 43 | 〃 | 〃 | 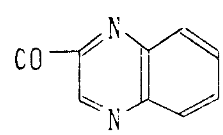 | |

to be continued

| | | | | |
|---|---|---|---|---|
| 44 | 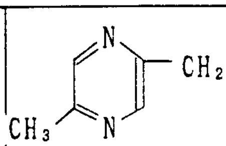 | -CH=CH-CH=CH | 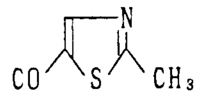 | |
| 45 | 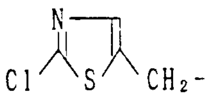 | 〃 | 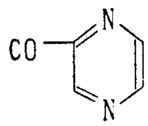 | [173 - 175 dec] |
| 46 | 〃 | 〃 | 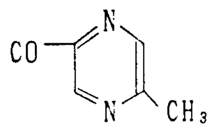 | |
| 47 | 〃 | 〃 | 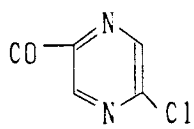 | |
| 48 | 〃 | 〃 | 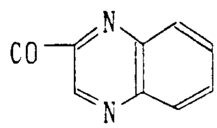 | |
| 49 | 〃 | 〃 | 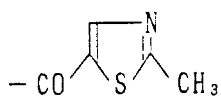 | |
| 50 | 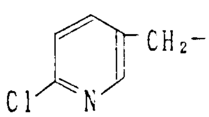 | $-CH=\overset{CH_3}{\overset{\mid}{C}}-CH=CH$ | 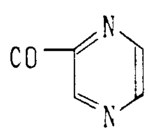 | |
| 51 | 〃 | 〃 | 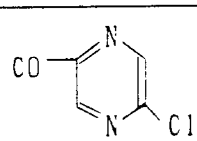 | |
| 52 | 〃 | 〃 | 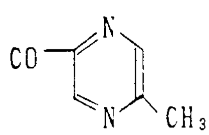 | |

to be continued

| | | | | |
|---|---|---|---|---|
| 53 | 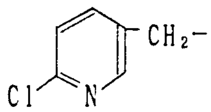 | 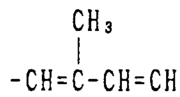 | 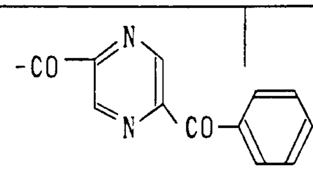 | |
| 54 | 〃 | 〃 | 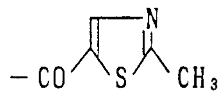 | |
| 55 | 〃 | 〃 | 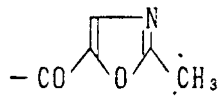 | |
| 56 | 〃 | 〃 | 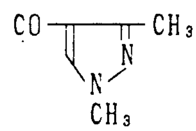 | |
| 57 | 〃 | 〃 | 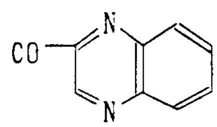 | |
| 58 | 〃 | 〃 | 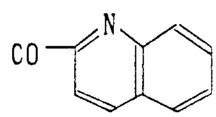 | |
| 59 | 〃 | 〃 | 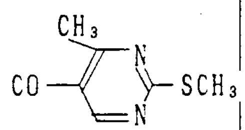 | |
| 60 | | $-CH=CH-S$ | $-CHO$ | |
| 61 | 〃 | 〃 | $COCH_3$ | |
| 62 | 〃 | 〃 | $COCH_2OCH_3$ | |
| 63 | 〃 | 〃 | $COCH_2Cl$ | |

to be continued

| | | | | |
|---|---|---|---|---|
| 64 | 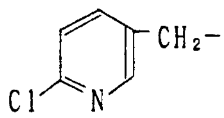 | $-CH=CH-S$ | $COC_2H_5$ | |
| 65 | 〃 | 〃 | 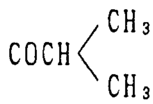 | |
| 66 | 〃 | 〃 | $COC_3H_7(n)$ | |
| 67 | 〃 | 〃 | $COCH=CH_2$ | |
| 68 | 〃 | 〃 | 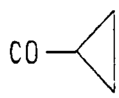 | |
| 69 | 〃 | 〃 | 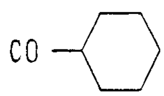 | |
| 70 | 〃 | 〃 | 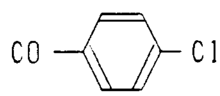 | |
| 71 | 〃 | 〃 | 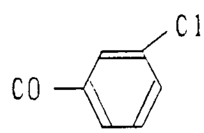 | |
| 72 | 〃 | 〃 | 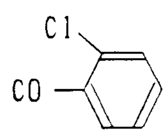 | |
| 73 | 〃 | 〃 | 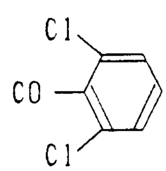 | |

to be continued

| | | | | |
|---|---|---|---|---|
| 74 | 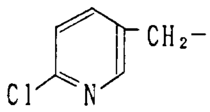 | −CH=CH−S | 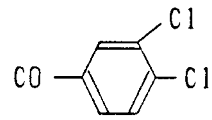 | |
| 75 | 〃 | 〃 | 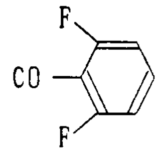 | |
| 76 | 〃 | 〃 | 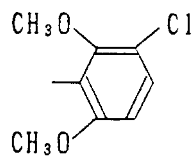 | |
| 77 | 〃 | 〃 | 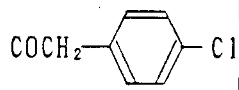 | |
| 78 | 〃 | 〃 | 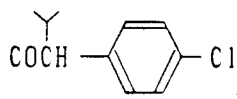 | |
| 79 | 〃 | 〃 | 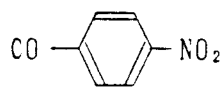 | |
| 80 | 〃 | 〃 | 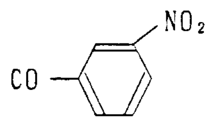 | |
| 81 | 〃 | 〃 | 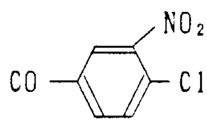 | |
| 82 | 〃 | 〃 | 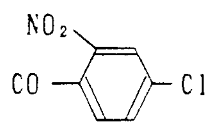 | |
| 83 | 〃 | 〃 | $SO_2CH_3$ | [167−168] |

to be continued

| | | | | |
|---|---|---|---|---|
| 84 | 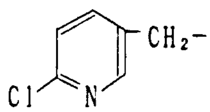 | $-CH=CH-S$ | $SO_2CH_2Cl$ | |
| 85 | 〃 | 〃 | $SO_2C_2H_5$ | |
| 86 | 〃 | 〃 | $SO_2CH_2CF_3$ | |
| 87 | 〃 | 〃 | 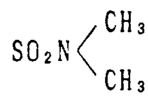 | |
| 88 | 〃 | 〃 | $SO_2CF_3$ | |
| 89 | 〃 | 〃 | 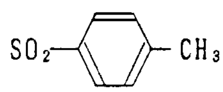 | |
| 90 | 〃 | 〃 | 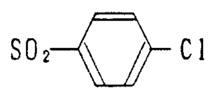 | |
| 91 | 〃 | 〃 | 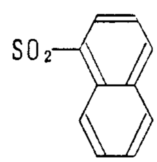 | |
| 92 | 〃 | 〃 | 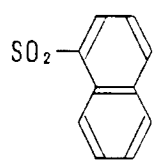 | |
| 93 | 〃 | 〃 | $CONHCH_3$ | [95−97] |

to be continued

| | | | | |
|---|---|---|---|---|
| 94 | 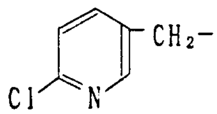 | $-CH=CH-S$ | $CONHC_2H_5$ | |
| 95 | 〃 | 〃 | $CONHCH_2CH=CH_2$ | |
| 96 | 〃 | 〃 | $CONHC_4H_9(t)$ | |
| 97 | 〃 | 〃 | CONH 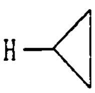 | |
| 98 | 〃 | 〃 | CONH 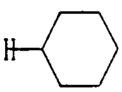 | |
| 99 | 〃 | 〃 | CONH 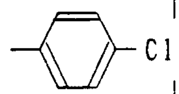 | [153 - 154] |
| 100 | 〃 | 〃 | CONH 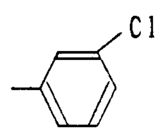 | |
| 101 | 〃 | 〃 | CONH 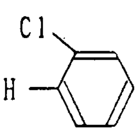 | |
| 102 | 〃 | 〃 | CONH 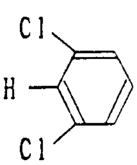 | (70-71) |
| 103 | 〃 | 〃 | $CSNHCH_3$ | |

to be continued

| | | | | |
|---|---|---|---|---|
| 104 | 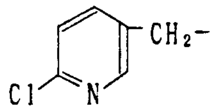 | $-CH=CH-S$ | $CSNHC_2H_5$ | |
| 105 | 〃 | 〃 | $CSNHCH_2CH=CH_2$ | |
| 106 | 〃 | 〃 | CSNH 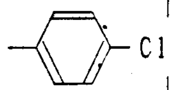 | |
| 107 | 〃 | 〃 | CSNH 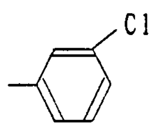 | |
| 108 | 〃 | 〃 | CSNH 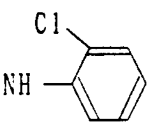 | |
| 109 | 〃 | 〃 | $CSNHCOOC_2H_5$ | |
| 110 | 〃 | 〃 | $COOC_2H_5$ | |
| 111 | 〃 | $CH_3$<br>$-C=CH=S$ | CO 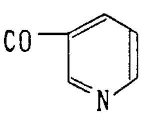 | |
| 112 | 〃 | 〃 | CO 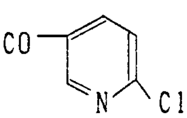 | |
| 113 | 〃 | 〃 | CO 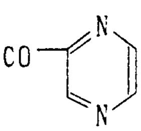 | 〔153-156dec〕 |

to be continued

| | | | | |
|---|---|---|---|---|
| 114 | 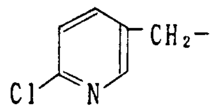 | CH₃<br>\|<br>− C=CH=S | 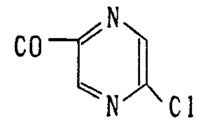 | |
| 115 | 〃 | 〃 | 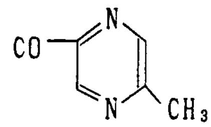 | [185-<br>188dec] |
| 116 | 〃 | CH₃ CH₃<br>\| \|<br>− C ═ C-S | 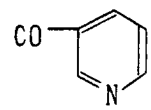 | |
| 117 | 〃 | 〃 | 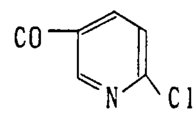 | |
| 118 | 〃 | 〃 | 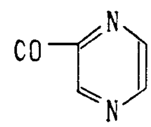 | [193-<br>195dec] |
| 119 | 〃 | 〃 | 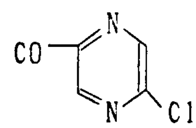 | |
| 120 | 〃 | 〃 | 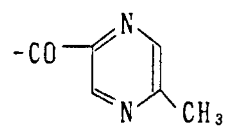 | |
| 121 | 〃 | CH₃<br>\|<br>-CH=C-S | 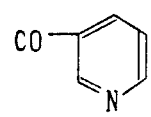 | |
| 122 | 〃 | 〃 | 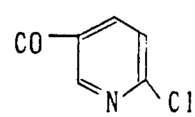 | |
| 123 | 〃 | 〃 | 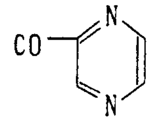 | [210℃ up] |

to be continued

| | | | | |
|---|---|---|---|---|
| 124 | 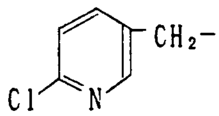 | 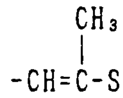 | 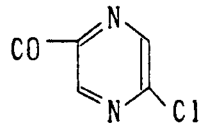 | |
| 125 | 〃 | 〃 | 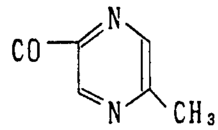 | |
| 126 | 〃 | 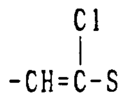 | 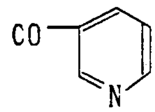 | |
| 127 | 〃 | 〃 | 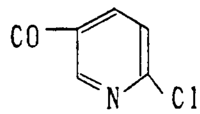 | |
| 128 | 〃 | 〃 | 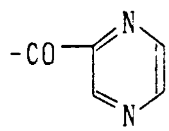 | |
| 129 | 〃 | 〃 | 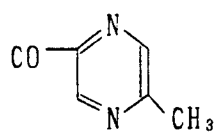 | |
| 130 | 〃 | 〃 | 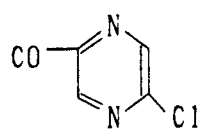 | |
| 131 | 〃 | - CH= CH- S | 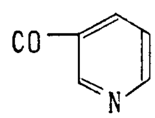 | [151-153] |
| 132 | 〃 | 〃 | 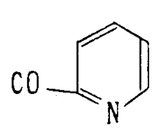 | |

to be continued

| | | | | |
|---|---|---|---|---|
| 133 | 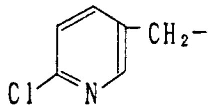 | −CH=CH−S | 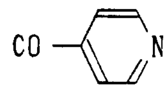 | [164−165] |
| 134 | 〃 | 〃 | 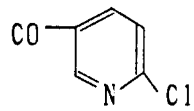 | [157 −158] |
| 135 | 〃 | 〃 | 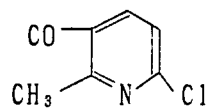 | |
| 136 | 〃 | 〃 | 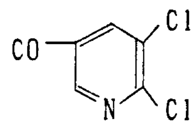 | |
| 137 | 〃 | 〃 | 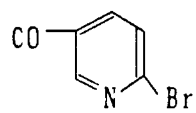 | |
| 138 | 〃 | 〃 | 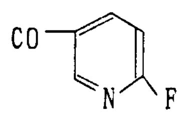 | |
| 139 | 〃 | 〃 | 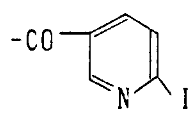 | |
| 140 | 〃 | 〃 | 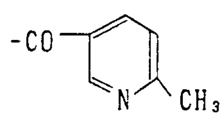 | |
| 141 | 〃 | 〃 | 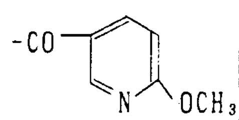 | |
| 142 | 〃 | 〃 | 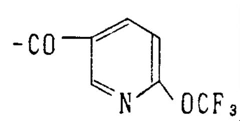 | |

to be continued

| | | | | |
|---|---|---|---|---|
| 143 | 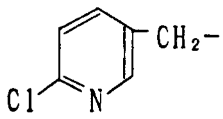 | $-CH=CH-S$ | 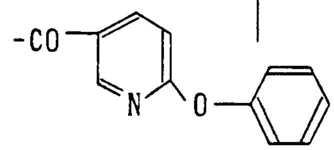 | |
| 144 | 〃 | 〃 | 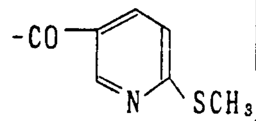 | |
| 145 | 〃 | 〃 | 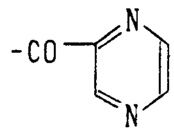 | [220° up] |
| 146 | 〃 | 〃 | 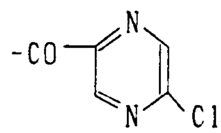 | [186-187] |
| 147 | 〃 | 〃 | 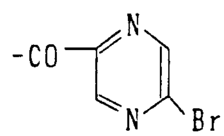 | |
| 148 | 〃 | 〃 | 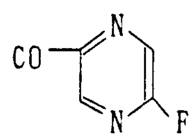 | |
| 149 | 〃 | 〃 | 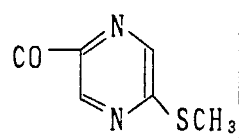 | |
| 150 | 〃 | 〃 | 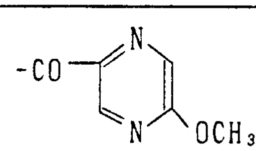 | |
| 151 | 〃 | 〃 | 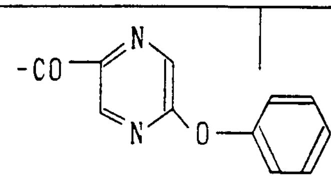 | |

to be continued

| | | | | |
|---|---|---|---|---|
| 152 | 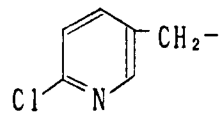 | $-CH=CH-S$ | 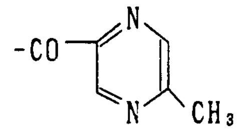 | [205－206] |
| 153 | 〃 | 〃 | 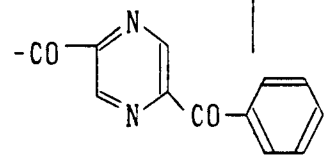 | |
| 154 | 〃 | 〃 | 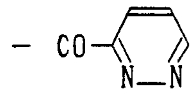 | |
| 155 | 〃 | 〃 | 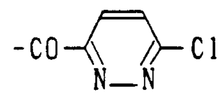 | |
| 156 | 〃 | 〃 | 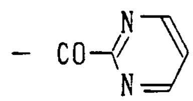 | |
| 157 | 〃 | 〃 | 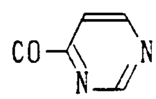 | |
| 158 | 〃 | 〃 | 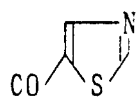 | |
| 159 | 〃 | 〃 | 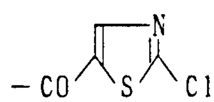 | |
| 160 | 〃 | 〃 | 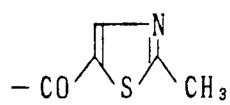 | [181 －183] |
| 161 | 〃 | 〃 | 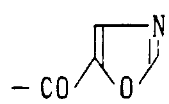 | |
| 162 | 〃 | 〃 | 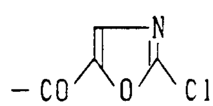 | |

to be continued

| | | | | |
|---|---|---|---|---|
| 163 | 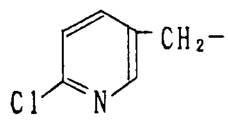 | $-CH=CH-S$ | 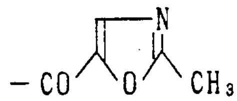 | |
| 164 | 〃 | 〃 | 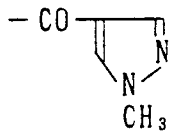 | |
| 165 | 〃 | 〃 | 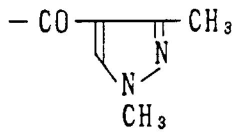 | |
| 166 | 〃 | 〃 | 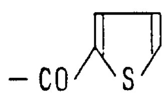 | |
| 167 | 〃 | 〃 | 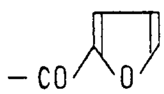 | |
| 168 | 〃 | 〃 | 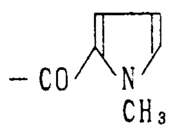 | |
| 169 | 〃 | 〃 | 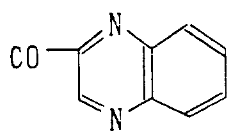 | |
| 170 | 〃 | 〃 | 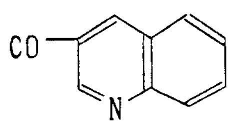 | |
| 171 | 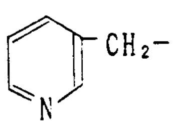 | 〃 | 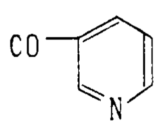 | |

to be continued

| | | | | |
|---|---|---|---|---|
| 172 | 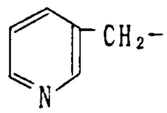 | -CH=CH-S | 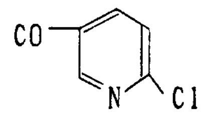 | |
| 173 | 〃 | 〃 | 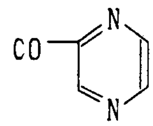 | |
| 174 | 〃 | 〃 | 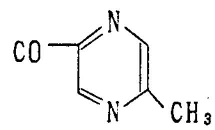 | |
| 175 | 〃 | 〃 | 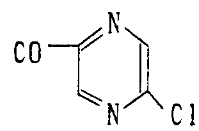 | |
| 176 | 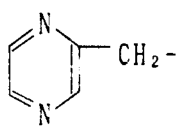 | 〃 | 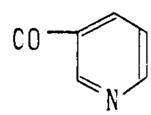 | |
| 177 | 〃 | 〃 | 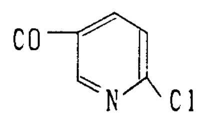 | |
| 178 | 〃 | 〃 | 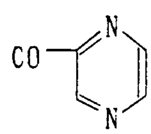 | |
| 179 | 〃 | 〃 | 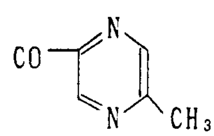 | |
| 180 | 〃 | 〃 | 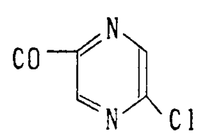 | |
| 181 | 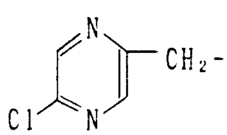 | 〃 | 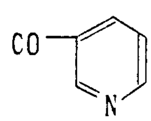 | |

to be continued

| | | | | |
|---|---|---|---|---|
| 182 | 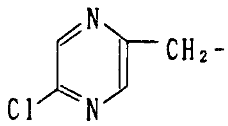 | − CH═CH− S | 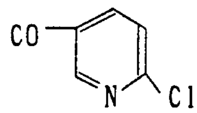 | |
| 183 | 〃 | 〃 | 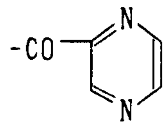 | |
| 184 | 〃 | 〃 | 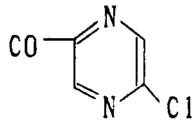 | |
| 185 | 〃 | 〃 | 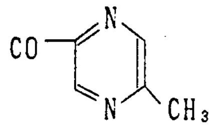 | |
| 186 | 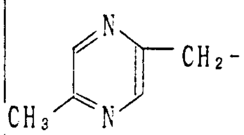 | 〃 | 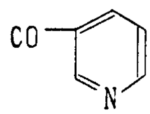 | |
| 187 | 〃 | 〃 | 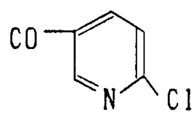 | |
| 188 | 〃 | 〃 | 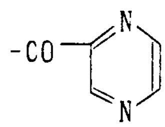 | |
| 189 | 〃 | 〃 | 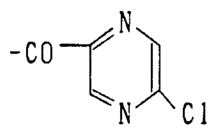 | |
| 190 | 〃 | 〃 | 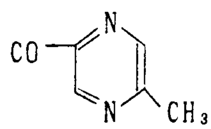 | |
| 191 | 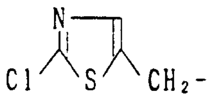 | 〃 | 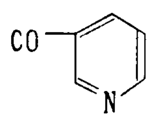 | |

to be continued

| | | | | |
|---|---|---|---|---|
| 192 | 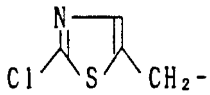 | − CH═CH − S | 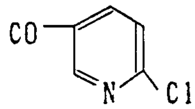 | |
| 193 | 〃 | 〃 | 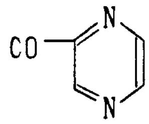 | [155−157dec] |
| 194 | 〃 | 〃 | 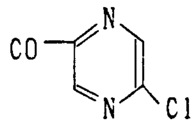 | |
| 195 | 〃 | 〃 | 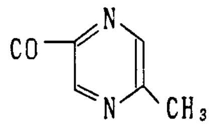 | |
| 196 | 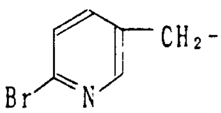 | 〃 | 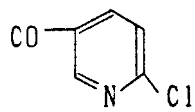 | |
| 197 | 〃 | 〃 | 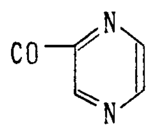 | |
| 198 | 〃 | 〃 | 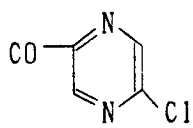 | |
| 199 | 〃 | 〃 | 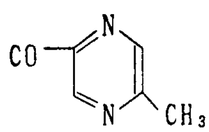 | |
| 200 | 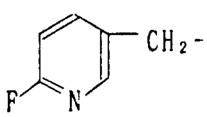 | 〃 | 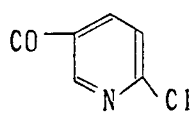 | |
| 201 | 〃 | 〃 | 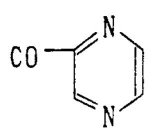 | |

to be continued

| | | | | |
|---|---|---|---|---|
| 202 | 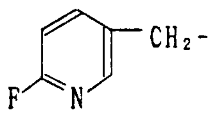 | $-CH=CH-S$ | 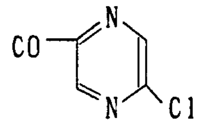 | |
| 203 | 〃 | 〃 | 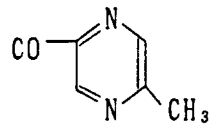 | |
| 204 | 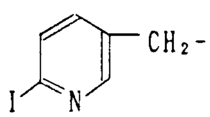 | 〃 | 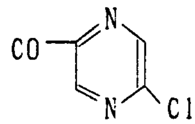 | |
| 205 | 〃 | 〃 | 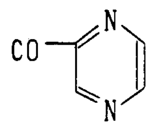 | |
| 206 | 〃 | 〃 | 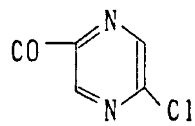 | |
| 207 | 〃 | 〃 | 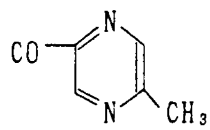 | |
| 208 | 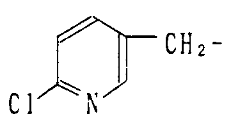 | $-CH=CH-CH_2$ | 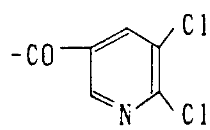 | |
| 209 | 〃 | 〃 | 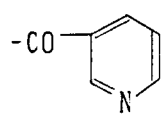 | |
| 210 | 〃 | 〃 | 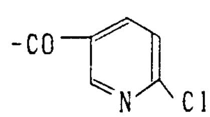 | |
| 211 | 〃 | 〃 | 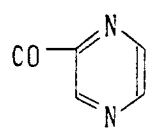 | |

to be continued

| | | | | |
|---|---|---|---|---|
| 212 | 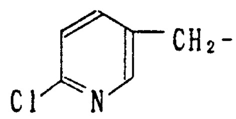 | $-CH=CH-CH_2$ | 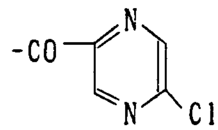 | |
| 213 | 〃 | 〃 | 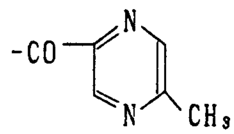 | |
| 214 | 〃 | 〃 | 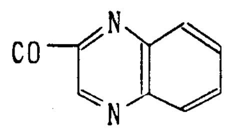 | |
| 215 | 〃 | 〃 | 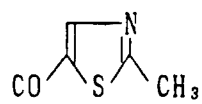 | |
| 216 | 〃 | 〃 | $CSNHCOOC_2H_5$ | |
| 217 | 〃 | 〃 | $SO_2CH_3$ | |
| 218 | 〃 | $-CH=CH-NH$ | 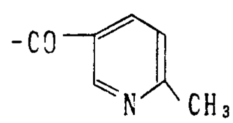 | |
| 219 | 〃 | 〃 | 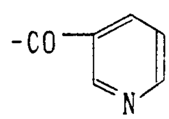 | |
| 220 | 〃 | 〃 | 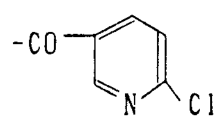 | |
| 221 | 〃 | 〃 | 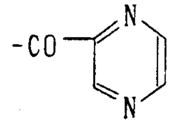 | |

to be continued

| | | | | |
|---|---|---|---|---|
| 222 | 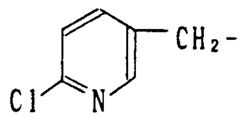 | $-CH=CH-NH$ | 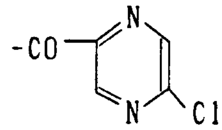 | |
| 223 | 〃 | 〃 | 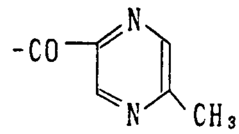 | |
| 224 | 〃 | 〃 | 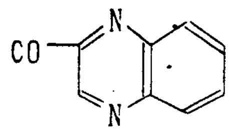 | |
| 225 | 〃 | 〃 | 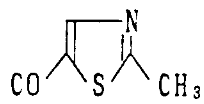 | |
| 226 | 〃 | 〃 | $CSNHCOOC_2H_5$ | |
| 227 | 〃 | 〃 | $SO_2CH_3$ | |
| 228 | 〃 | $-CH=CH-CH=N$ | 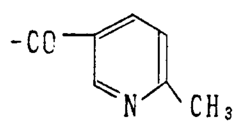 | |
| 229 | 〃 | 〃 | 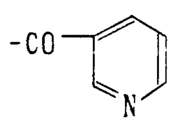 | |
| 230 | 〃 | 〃 | 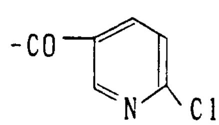 | |
| 231 | 〃 | 〃 | 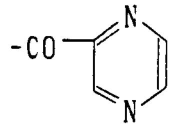 | |

to be continued

| | | | | |
|---|---|---|---|---|
| 232 | 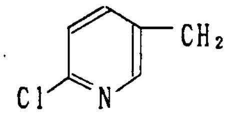 | — CH=CH-CH=N | 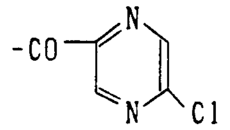 | |
| 233 | 〃 | 〃 | 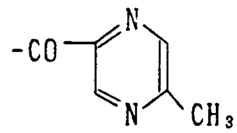 | |
| 234 | 〃 | 〃 | 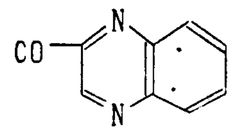 | |
| 235 | 〃 | 〃 | 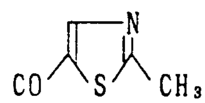 | |
| 236 | 〃 | 〃 | $CSNHCOOC_2H_5$ | |
| 237 | 〃 | 〃 | $SO_2CH_3$ | |
| 238 | 〃 | $-CH=CH-CH_2-S$ | 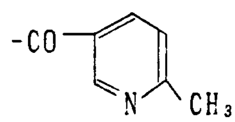 | |
| 239 | 〃 | 〃 | 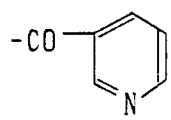 | |
| 240 | 〃 | 〃 | 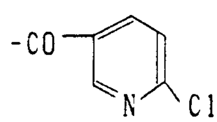 | |
| 241 | 〃 | 〃 | 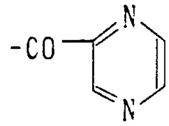 | |

to be continued

| | | | | |
|---|---|---|---|---|
| 242 | 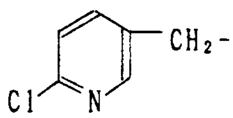 | $-CH=CH-CH_2-S$ | 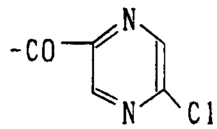 | |
| 243 | " | " | 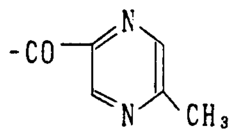 | |
| 244 | " | " | 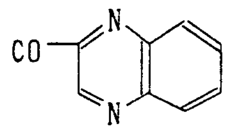 | |
| 245 | " | " | 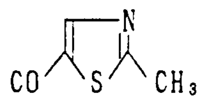 | |
| 246 | " | " | $CSNHCOOC_2H_5$ | |
| 247 | " | " | $SO_2CH_3$ | |
| 248 | " | $-CH=CH-CH_2-O$ | 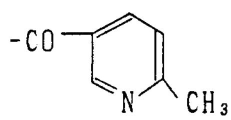 | |
| 249 | " | " | 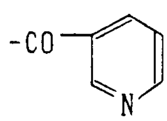 | |
| 250 | " | " | 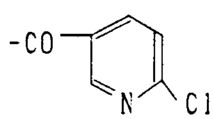 | |
| 251 | " | " | 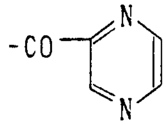 | |

to be continued

| | | | | |
|---|---|---|---|---|
| 252 | 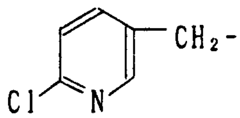 | -CH=CH-$CH_2$-O | 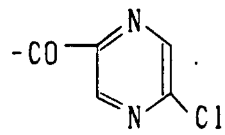 | |
| 253 | 〃 | 〃 | 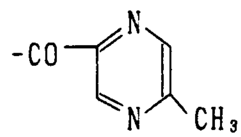 | |
| 254 | 〃 | 〃 | 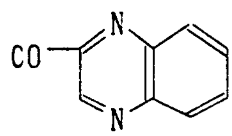 | |
| 255 | 〃 | 〃 | 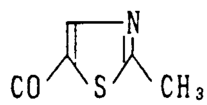 | |
| 256 | 〃 | 〃 | $CSNHCOOC_2H_5$ | |
| 257 | 〃 | 〃 | $SO_2CH_3$ | |
| 258 | 〃 | − CH=CHO | 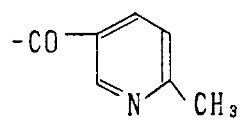 | |
| 259 | 〃 | 〃 | 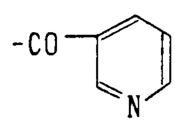 | |
| 260 | 〃 | 〃 | 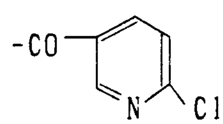 | |
| 261 | 〃 | 〃 | 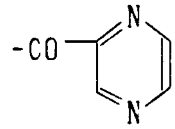 | |

to be continued

| | | | | |
|---|---|---|---|---|
| 262 | 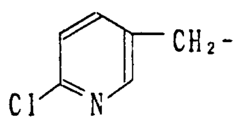 | $-CH=CHO$ | 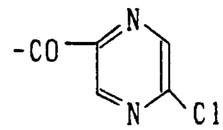 | |
| 263 | 〃 | 〃 | 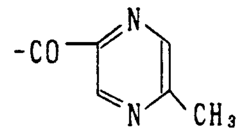 | |
| 264 | 〃 | 〃 | 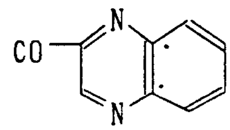 | |
| 265 | 〃 | 〃 | 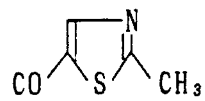 | |
| 266 | 〃 | 〃 | $CSNHCOOC_2H_5$ | |
| 267 | 〃 | 〃 | $SO_2CH_3$ | |
| 268 | 〃 | $-CH=CH-S$ | 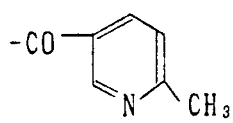 | |
| 269 | 〃 | 〃 | 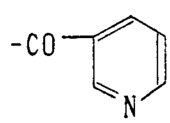 | |
| 270 | 〃 | 〃 | 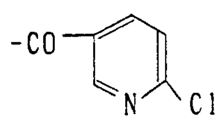 | |
| 271 | 〃 | 〃 | 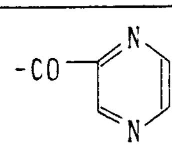 | [163－165] |

to be continued

| | | | | |
|---|---|---|---|---|
| 272 | 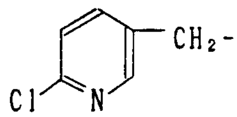 | $-CH=CH-S$ | 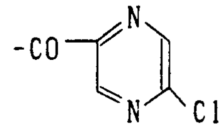 | |
| 273 | 〃 | 〃 | 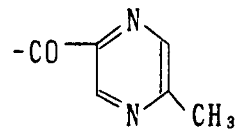 | |
| 274 | 〃 | 〃 | 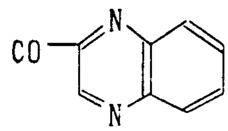 | |
| 275 | 〃 | 〃 | 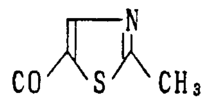 | |
| 276 | 〃 | 〃 | $CSNHCOOC_2H_5$ | |
| 277 | 〃 | 〃 | $SO_2CH_3$ | |
| 278 | 〃 | 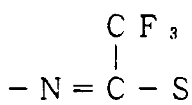 | 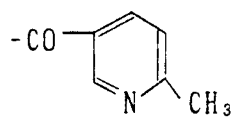 | |
| 279 | 〃 | 〃 | 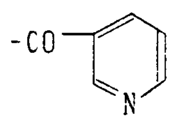 | |
| 280 | 〃 | 〃 | 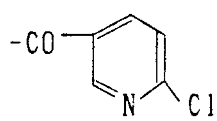 | |
| 281 | 〃 | 〃 | 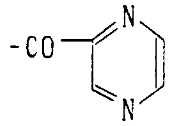 | [109－111] |

to be continued

| | | | | |
|---|---|---|---|---|
| 282 | 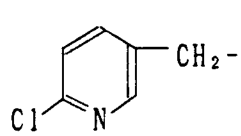 | 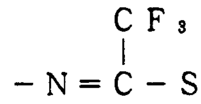 | 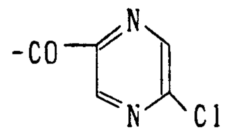 | |
| 283 | 〃 | 〃 | 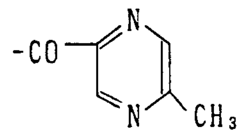 | |
| 284 | 〃 | 〃 | 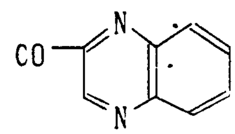 | |
| 285 | 〃 | 〃 | 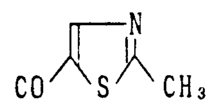 | |
| 286 | 〃 | 〃 | $CSNHCOOC_2H_5$ | |
| 287 | 〃 | 〃 | $SO_2CH_3$ | |
| 288 | 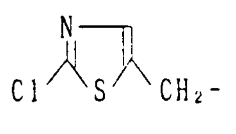 | 〃 | 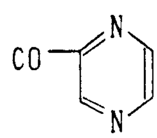 | [138－139] |

An insecticide specified in the present invention contains a compound having the general formula [I] as an active ingredient. Although this compound can be used without formulating, it is normally used in a typical form for agricultural chemicals, i.e. wettable powder, water-soluble powder, dust, emulsifiable concentrate, granules, flowable, smoke generator, fumigant and the like. For additives and carriers, when using them for solid formulation, plant-origin powder such as soybean powder and wheat flour; mineral fine powder such as diatomaceous earth, apatite, gypsum, talc, bentonite and clay; and organic and inorganic compounds such as sodium benzoate, urea and Glauber's salt can be used.

For a liquid formulation, vegetable oil; mineral oil; distillated fractions of petroleum such as kerosene, xylene and solvent naphtha; cyclohexane; cyclohexanone; dimethylformamide; dimethylsulfoxide; trichloroethylene; methylisobutylketone; water and the like can be used as a solvent. In the formulations described above, if necessary, a surfactant can be added thereto in order to give homogeneous and stabilized dilution. The wettable powder, the emulsifiable concentrate, the water-soluble powder and the flowable prepared as described above can be used after diluting to the prescribed concentrations of suspension or emulsion in water, whereas the dust and the granules can be used directly without diluting by means of spraying plant crops.

Although the compounds specified in the present invention are, of course, sufficiently effective even in single use, they can be also used as the mixture with various insecticides, acaricides and fungicides.

Representative acaricides and insecticides which can be used as a mixture with the compound specified in the present invention include the following.

Acaricides (Fungicides):

Chorobenzylate, chloropropylate, proclonol, phenisobromolate, dicofol, dinobuton, binapacryl, chlorphenamidine, amitraz, BPPS, PPPS, benzomate, hexythiazox, tenbutatin oxide, polynactin, quinomethionate, thioquinox, CPCBS, tetradifon, kayacide, avermectin, chlofentezin, flubenzimin, flufenoxuron, BCPE, cyhexatin, pyridaben, fenproximate, fenezaquin, thiphanate methyl, benomyl, thiuram, IBP, EDDP, phtalide, probenazole, isoprothiolane, TPN, captan, polyoxin, blastcidin-S, kasugamycin, validamycin, tricyclazole, pyroquilon, phenazin oxide, mepronil, flutolanil, pencycuron, iprodione, hymexazol, metalaxyl, triflumizol, diclomezine, tecloftalam, vinclozolin, procymidone, bitertanol, triadimefon, procloraz, pirifenox, fenarimol, fenpropimorph, trifoline, oxycarboxin, pefrazoate, diclomezine, fluazinam, oxadixyl, ethoavinolac, TPTH, propamocarb, fosetyl, dihydrostreptomycin, anilazine, dithianon, diethofencarb;

Organophosphorous and carbamate insecticides (acaricides):

Fenthion, fenitrothion, diazinon, chlorpyriphos, ESP, vamidothion, fenthoate, dimethoate, formothion, malathion, dipterex, thiometon, phosmet, menazon, dichlorvos, acephate, EPBP, diaryfol, methyl parathion, oxydimeton methyl, ethion, pyrachlofos, monocrotophos, aldicarb, propoxur, methomyl, BPMC, MTMC, NAC, cartap, carbosulfan, benfuracarb, pirimicarb, ethiofencarb, fenoxycarb, thiodicarb, salithion, carbofuran, merthocarb;

Pyrethroid insecticides (acaricides):

Permethrin, cypermethrin, decamethrin, fenvalerate, fenpropathrin, pyrethrin, tetramethrin, resmethrin, dimethrin, propathrin, bifenthrin, prothrin, fivalinate, cyfluthrin, cyhalothrin, flucythrinate, ethofenprox, cycloprothrin, tralomethrin, silaneophene;

Benzoylureas and other insecticides:

Diflubenzuron, chlorfluazuron, triflumuron, teflubenzuron, buprofezin, pyriproxiphen, and machine oil.

The formulations comprising the compound of this invention are illustrated by the following Examples, however, a carrier and a surface active agent to be added to the formulation shall not be limited by the following Examples.

Example 3: Emulsifiable concentrate

| | |
|---|---|
| A compound of the invention | 10 parts |
| Alkyl phenylpolyoxyethylene | 5 parts |
| Dimethylformamide | 50 parts |
| Xylene | 35 parts |

Above components are mixed and dissolved to obtain the formulation. Upon use, the formulation is diluted with water to give the emulsuion thereof, then the emulsion is sprayed.

Example 4: Wettable powder

| | |
|---|---|
| A compound of the invention | 20 parts |
| Sulfate ester of higher alcohol | 5 parts |
| Diatomaceous earth | 70 parts |
| Artificial silicate | 5 parts |

Above components are mixed and micronized to obtain the formulation. Upon use, the formulation is diluted with water to give the suspension thereof, then the suspension is sprayed.

Example 5: Dust

| | |
|---|---|
| A compound of the invention | 5 parts |
| Talc | 94.7 parts |
| Artificial silicate | 0.3 parts |

Above components are mixed and pulverized to obtain the formulation. Upon use, the formulation is applied directly without diluting.

Example 6: Granular formulation

| | |
|---|---|
| A compound of the invention | 5 parts |
| Clay | 73 parts |
| Bentonite | 20 parts |
| Sodium dioctylsulfosuccinate | 1 part |
| Sodium phosphate | 1 part |

Above components are mixed and granulated to obtain the formulation. Upon use, the formulation is applied directly without diluting.

Industrial Application

Test Example 1: Insecticidal efficacy against cotton aphids

Cotton aphids were inoculated at the rate of 30-50 aphids per plot on 10 days old cucumber leaves planted in a pot with diameter of 10 cm by using a small brush. The aphids injured during the inoculation were removed one day later, then the solution of the compound adjusted to 125 ppm by the dilution of the emulsifiable concentrate prepared according to the Example 3 with water was sprayed. The aphids were placed in an incubator maintained at 25°C and 65% R.H., then the number of the living aphids was counted after 7 days to determine the control ratio of aphids by comparison with an untreated plot. The results are summarized in Table 2.

[Table 2]

| Compound No. | Comtrol Efficacy (7 days later) 125ppm (%) |
|---|---|
| 1 | 100 |
| 2 | 100 |
| 8 | 100 |
| 9 | 100 |
| 15 | 100 |
| 20 | 100 |
| 22 | 100 |
| 23 | 100 |
| 40 | 100 |
| 45 | 100 |
| 83 | 100 |
| 93 | 100 |
| 113 | 100 |
| 115 | 100 |
| 118 | 100 |
| 123 | 100 |
| 131 | 100 |
| 133 | 100 |
| 134 | 100 |
| 145 | 100 |
| 146 | 100 |
| 152 | 100 |
| 160 | 100 |
| 271 | 100 |
| Comparative Compound A | 0 |
| Comparative Compound B | 100 |

Comparative Compound A:

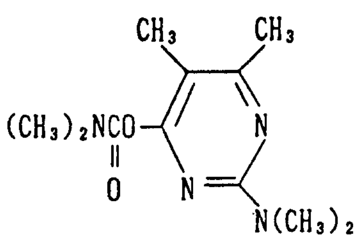

(pyrimicarb)

Comparative Compound B:

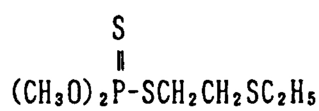

(thiometon)

Test Example 2: Insecticidal efficacy against planthoppers

Young rice plant seedlings in a stage of 7 days after the germination were dipped for 30 seconds into a solution of the compound in water with a concentration of the compound of 125 ppm prepared by the dilution of the emulsifiable concentrate prepared according to the protocol described in Example 3. After drying the seedlings were then put into a test tube and 10 third-instar larvae of a strain of planthoppers resistant against organophosphorous and carbamate insecticides were inoculated therein. The test tube was shielded with gauze and placed in an incubator maintained at 25 ° C and 65% R.H., and the mortality after 5 days was determined. The results were summarized in Table 3.

Table 3

| Compound No. | Mortality (5 days later) 125ppm (%) |
|---|---|
| 1 | 100 |
| 2 | 100 |
| 8 | 100 |
| 15 | 100 |
| 20 | 100 |
| 22 | 100 |
| 24 | 100 |
| 40 | 100 |
| 45 | 100 |
| 83 | 100 |
| 93 | 100 |
| 113 | 100 |
| 115 | 100 |
| 118 | 100 |
| 123 | 100 |
| 134 | 100 |
| 145 | 100 |
| 146 | 100 |
| 152 | 100 |
| 160 | 100 |
| 271 | 100 |
| Comparative Compound C | 0<br>0 |

Comparative Compound C :

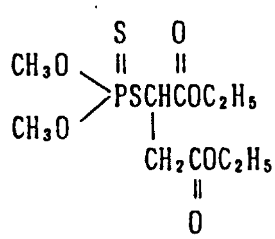

(malathion)

The compound specified in the present invention demonstrates excellent insecticidal activity on various species of pest insect such as the army worm, the diamondback moth, aphids, the green rice leafhopper and the brown rice planthopper. Recently, resistance to insecticides including organophosphorus and carbamate compounds has developped among various insect species such as the diamondback moth,

leafhopper, planthopper and aphids, resulting in a problem of insufficient efficacy of the insecticides described above against those insects. Therefore, an effective insecticide for those insecticide-resistant insect species are urgently required. The compounds specified in the present invention have excellent insecticidal efficacy not only against insecticide-sensitive pest insects but also against strains of pest insects resistant to insecticides comprising organophosphorus and carbamate compounds.

## Claims

1. A compounds having the general formula [I]:

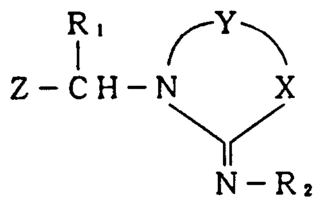

〔I〕

wherein
Z is an unsubstituted or substituted 5- or 6-membered heterocyclic group containing nitrogen;
X is a carbon, nitrogen, sulfur or oxygen atom;
Y is, when X is a carbon atom, (a) in a form of -Y-X;
(a)

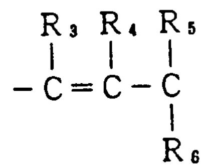

when X is a nitrogen atom, (b) or (c) in a form of -Y-X;
(b)

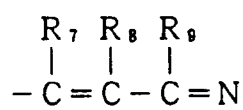

(c)

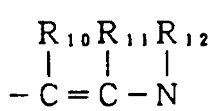

when X is a sulfur atom, (d), (e) or (f) in a form of -Y-X;
(d)

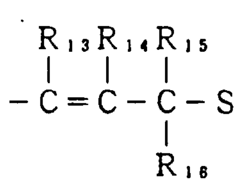

(e)

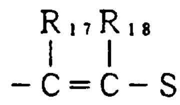

(f)

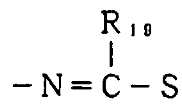

when X is an oxygen atom, (g) or (h) in a form of -Y-X;
(g)

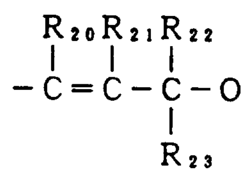

(h)

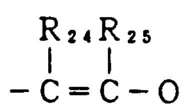 ;

$R_2$ is an electron attractive group other than nitro, cyano and trifluoroacetyl;
$R_1$ and $R_3$ ~ $R_{25}$ are independently a hydrogen atom, a halogen atom or an unsubstituted or substituted lower alkyl;
Or, Y is, when X is a carbon atom, (i) in a form of -Y-X;
(i)

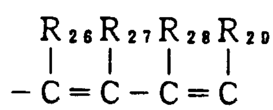 ;

$R_2$ is an unsubstituted or substituted heterocyle carbonyl, other than furancarbonyl, thiophenecarbonyl and pyridinecarbonyl;
$R_{26}$ ~ $R_{29}$ are independently a hydrogen atom, a halogen atom or an unsubstituted or substituted lower alkyl;
or its salt.

**2.** A process for preparing a compound represented by the general formula [I]:

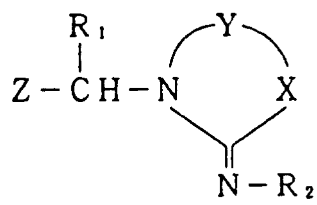

〔I〕

wherein Z, X, Y, $R_1$ and $R_2$ are as defined above, characterized in that a compound represented by the general formula [II]:

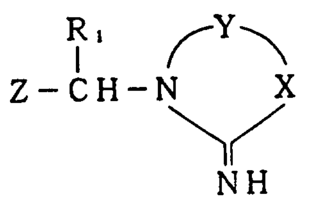

〔II〕

wherein Z, $R_1$, X and Y are as defined above, are reacted with a compound represented by the formula $R_2$-Hal, wherein Hal and $R_2$ are as defined above.

**3.** An insecticide characterized in that the insecticide comprises one or two or more compounds represented by the general formula [I]:

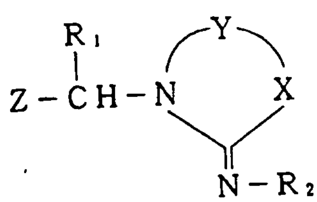

〔I〕

wherein Z, X, Y, $R_1$ and $R_2$ are as defined above, as effective ingredients.

# INTERNATIONAL SEARCH REPORT

International Application No PCT/JP92/00283

**I. CLASSIFICATION OF SUBJECT MATTER** (if several classification symbols apply, indicate all) [6]

According to International Patent Classification (IPC) or to both National Classification and IPC

Int. $Cl^5$ C07D213/81, C07D401/06, C07D413/06, C07D413/14, C07D417/06, C07D417/14, C07D401/14

**II. FIELDS SEARCHED**

Minimum Documentation Searched [7]

| Classification System | Classification Symbols |
|---|---|
| IPC | C07D213/81, C07D401/06, C07D401/14, C07D413/06, C07D413/14, C07D417/06, C07D417/14 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | US, A, 3,622,585 (Parke, Davis and Co.), November 23, 1971 (23. 11. 71) | 1-3 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| May 21, 1992 (21. 05. 92) | June 9, 1992 (09. 06. 92) |
| **International Searching Authority** | **Signature of Authorized Officer** |
| Japanese Patent Office | |